# EUROPEAN PATENT SPECIFICATION

(11) **EP 1 060 008 B1**
(45) Date of publication and mention of the grant of the patent: **24.11.2004**
(21) Application number: 99937888.8
(22) Date of filing: 26.02.1999
(51) Int. Cl.: B01J 2/04, A61K 9/16

(54) **METHOD OF PARTICLE FORMATION**
VERFAHREN ZUR HERSTELLUNG VON TEILCHEN
PROCEDE DE FORMATION DE PARTICULES

(30) Priority: 02.03.1998 GB 9804379
(43) Date of publication of application: 20.12.2000
(73) Proprietor: Nektar Therapeutics UK Limited, Bradford, West Yorkshire BD7 1HR (GB)
(72) Inventor: HANNA, Mazen, Hermiz, Bradford BD9 6PQ (GB); HUMPHREYS, Gwynfor Owen, Halifax HX2 7TU (GB); STOREY, Richard, Canterbury CT4 (GB); SHEKUNOV, Boris, Baildon, West Yorkshire BD17 5AL (GB)
(74) Representative: Brewster, Andrea Ruth
(86) International application number: PCT/GB1999/000587
(87) International publication number: WO 1999/044733

(56) References cited:
- WO-A-95/01221
- WO-A-95/21688
- WO-A-96/00610
- GB-A- 2 322 326

## Description

### Field of the Invention

This invention relates to the controlled formation of particulate products using supercritical fluids. It provides a method for the formation of substances in particulate form, and also a particulate product of the method.

### Background to the Invention

It is known to form particles of a substance of interest by dissolving or suspending it in a suitable vehicle and then using a supercritical fluid to extract the vehicle, under supercritical conditions, to cause precipitation of fine particles.

One particular technique for doing this is known as "SEDS" (Solution Enhanced Dispersion by Supercritical fluids). This is described in WO-95/01221 and (in a modified form) in WO-96/00610. The essence of SEDS is that a solution or suspension of a substance of interest, in an appropriate vehicle, is co-introduced into a particle formation vessel with a supercritical fluid, in such a way that dispersion and extraction of the vehicle occur substantially simultaneously by the action of the supercritical fluid, and substantially immediately on introduction of the fluids into the vessel. The pressure and temperature inside the vessel are carefully controlled during this process.

SEDS allows a high degree of control over conditions such as pressure, temperature and fluid flow rates, and over the physical dispersion of the solution/suspension, at the exact point where particle formation occurs (ie at the point where the vehicle is extracted into the supercritical fluid). It therefore allows excellent control over the size, shape and other physicochemical properties of the particles formed.

SEDS also allows particles to be formed very quickly, and indeed can be so effective that under some conditions, particle formation is a little *too* rapid and blockages can occur in the system. Typically, blockages occur at the point where the fluids meet and enter the particle formation vessel, which is also the point of particle formation. The fluids are introduced through a suitable inlet means, typically a nozzle, and it is here that blockages occur.

The supercritical fluid most commonly used to extract the vehicle is supercritical carbon dioxide, due to its relatively low cost, its non-toxicity and its convenient critical temperature and pressure values. However, in many cases carbon dioxide is such an effective extractor that again, it leads to rapid particle formation and blockages.

If a fluid inlet such as a nozzle becomes blocked, pressure quickly builds up upstream of the point of entry of the fluids into the particle formation vessel. Pumping of the fluids tends, however, to continue until either the blockage clears or the system over-pressurises, leading to the pumps cutting out and the whole process being aborted.

This problem can in part be overcome by altering the operating conditions. For instance, one might use a more dilute solution of the substance of interest, a higher flow rate for that solution (relative to that of the supercritical fluid), a lower flow rate for the supercritical fluid, etc.. These modifications can be effective, but not in all cases and not always to a satisfactory extent. Moreover, lowering the supercritical fluid flow rate can unduly affect the characteristics (in particular, size control) of the particles formed.

The present invention aims to overcome or at least mitigate the problem, and hence improve upon some of the current supercritical fluid particle formation techniques, in particular SEDS. It thus aims to extend the useful applications of supercritical fluid particle formation technology.

### Statements of the Invention

According to a first aspect of the present invention, there is provided a method for forming particles of a substance, the method comprising:-
(a) preparing a solution or suspension of the substance in a vehicle;
(b) introducing the solution or suspension into a particle formation vessel via a fluid inlet means; and
(c) introducing a primary supercritical fluid, capable of acting as an anti-solvent for the substance, into the particle formation vessel, under conditions which allow the supercritical fluid to extract the vehicle from the solution or suspension and hence cause the formation of particles of the substance;
characterised in that a secondary fluid is introduced into the particle formation vessel in the flow of the primary supercritical fluid, upstream of the point of contact between the primary supercritical fluid and the solution or suspension, the secondary fluid having a lower capacity for extracting the vehicle than that of the primary supercritical fluid.

In the method of the invention, the secondary fluid effectively acts as a "diluent" for the other fluids. Its presence slows the rate of particle formation and so can help prevent blockage of the fluid inlet means. Particle formation can thus be "postponed", by an amount sufficient for it to occur just downstream of the fluid inlet means, so that the particles formed cannot create blockages.

Provided the secondary fluid is used in a suitable amount, it does not seem to interfere unduly with the nature of the particle formation process - in other words, one can still achieve good control over the characteristics of the particles formed. Indeed, the secondary fluid can actually provide enhanced dispersion of the solution or suspension, and hence a better product.

The method of the invention can be used in any particle formation situation where nucleation and precipitation, occur too quickly, for instance when the chosen vehicle is highly soluble in the chosen primary supercritical fluid, or at high concentrations of the target substance in the solution or suspension.

The method of the invention is particularly useful when the particles are formed using the SEDS process; indeed it can improve upon, whilst retaining all the usual advantages of, SEDS.

Accordingly, most of the technical features of SEDS, as disclosed in WO-95/01221 and WO-96/00610, apply also to the present invention. The technical information contained in the earlier publications, as to the execution of SEDS, can also be applicable when carrying out the present invention.

In the following description, the term "supercritical fluid" means a fluid substantially at or above its critical pressure (P_{c}) and critical temperature (T_{c}) simultaneously. In practice, the pressure of the fluid is likely to be in the range between 1.01 and 7.0 of its critical pressure, and its temperature in the range between 1.01 and 7.0 of its critical temperature (in Kelvin).

The term "vehicle" means a fluid which is able to carry a solid or solids in solution or suspension. A vehicle may be composed of one or more component fluids. The vehicle used in the present invention should be substantially soluble in the chosen primary supercritical fluid, to allow its extraction at the point of particle formation.

The term "supercritical solution" means one or more supercritical fluids together with one or more vehicles which it or they have extracted and dissolved. The solution should still itself be in the supercritical state, at least within the particle formation vessel.

The terms "disperse" and "dispersion" refer to the formation of droplets, or of other analogous fluid elements, of the solution or suspension and/or of the vehicle.

The substance to which the method of the invention is applied may be any substance which needs to be produced in particulate form. It may be a substance for use in or as a pharmaceutical. However, the particulate product may also be a product of use in the ceramics, explosives or photographic industries; a foodstuff; a dye; a coating; etc. It may be organic or inorganic, monomeric or polymeric. In each case, the principle behind the method of the invention remains the same; the technician need only adjust operating conditions in order to effect proper control over the particles being formed.

The substance may be in a single or multi-component form - it could for instance comprise an intimate mixture of two materials, or one material in a matrix of another, or one material coated onto a substrate of another, or other similar mixtures. The particulate product, formed from the substance using the method of the invention, may also be in a multi-component form - such products may be made from solutions or suspensions containing only single component starting materials, provided the solutions/suspensions are introduced with the primary and secondary fluids in the correct manner (more than one solution/suspension may be introduced into the particle formation vessel with the primary supercritical fluid).

The particulate product may also be a substance formed from an in situ reaction (ie, immediately prior to, or on, the solution/suspension contacting the primary supercritical fluid) between two or more reactant substances, each carried by an appropriate vehicle. Such modifications, involving the use of in situ reactions and/or more than one solution or suspension of a substance of interest, are described in connection with SEDS in WO-95/0221 and WO-96/00610, and can also be applied when carrying out the present invention.

The primary supercritical fluid may be any suitable supercritical fluid, for instance supercritical carbon dioxide, nitrogen, nitrous oxide, sulphur hexafluoride, xenon, ethylene, chlorotrifluoromethane, ethane or trifluoromethane. A particularly preferred supercritical fluid is supercritical carbon dioxide, due to its relatively low cost, toxicity, flammability and critical temperature.

The primary supercritical fluid may optionally contain one or more modifiers, for example methanol, ethanol, isopropanol or acetone. When used, a modifier preferably constitutes not more than 20%, and more preferably between 1% and 10%, of the molar volume of the supercritical fluid. The term "modifier" is itself well known to those skilled in the art. A modifier (or co-solvent) may be described as a chemical which, when added to a supercritical fluid, changes the intrinsic properties of the fluid at or around its critical point.

The vehicle may be any appropriate fluid which either dissolves or suspends the substance of interest and is itself substantially soluble in the chosen primary supercritical fluid (but less soluble in the secondary fluid). The choice of vehicle in any particular case will depend on the nature of the substance, on the primary supercritical fluid, on the secondary fluid and on other practical criteria including those governing the desired end product. The term "vehicle" encompasses a mixture of two or more fluids which together have the necessary characteristics vis-a-vis the substance of interest and the other fluids involved.

The choice of a suitable combination of primary supercritical fluid, modifier (where desired), secondary fluid and vehicle for any desired product will be well within the capabilities of a person of ordinary skill in the art.

The primary supercritical fluid must be capable of acting as an anti-solvent for the substance. It must therefore be miscible with the chosen vehicle so that it can extract the vehicle from the solution or suspension, but it must not at that point extract or dissolve the substance itself as the particles are formed. In other words, it must be chosen so that the substance is for all practical purposes insoluble in it.

The choice of suitable operating conditions so as to allow extraction and particle formation to occur will be well within the capabilities of the person skilled in this art. Generally, the conditions in the particle formation vessel must be such that the primary supercritical fluid, and the supercritical solution which is formed when it extracts the vehicle, both remain in the supercritical form whilst in the vessel. Extraction of the vehicle by the primary supercritical fluid is then effectively immediate when the solution/suspension and the primary supercritical fluid come into contact (which is preferably, as in the SEDS process, at the point where both fluids enter the particle formation vessel). This allows the rapid formation of pure, dry particulate products. The exact pressures and temperatures needed to achieve this situation depend of course on the nature of the primary supercritical fluid and on the substance, the vehicle and other fluids being used.

The secondary fluid is chosen primarily so as to have a lower capacity for the chosen vehicle than does the primary supercritical fluid, by which is meant that the vehicle is less soluble in the secondary than in the primary fluid under the operating conditions (in particular temperature and pressure) being used. This is so that the secondary fluid does not to any significant extent extract the vehicle from the solution or suspension - it is then only present to slow the rate of particle formation, and does not interfere with the nature of that particle formation.

The vehicle extractive capacity of the secondary fluid is preferably less than half as great as that of the primary supercritical fluid, more preferably less than 30% as great, most preferably less than 20% as great, such as around 10% as great. Vehicle extractive capacities of even less than 10% of that of the primary supercritical fluid can sometimes be achieved, for instance when using a noble gas such as helium as the secondary fluid.

Even more preferably, the secondary fluid has almost no capacity at all for dissolving the chosen vehicle.

The secondary fluid should also be inert with respect to the substance and the vehicle, again so as not to interfere with the particulate product.

The secondary fluid is typically itself a supercritical fluid (especially bearing in mind the operating conditions in which it is co-introduced with the other fluids), however it need not necessarily be so. It could for instance be a liquid which is fully miscible with the primary supercritical fluid and thus forms with the primary supercritical fluid a solution having, overall, a lower extractive capacity for the vehicle than that of the primary fluid alone.

Supercritical nitrogen is a particularly useful secondary fluid, especially where the substance has been dissolved or suspended in one or more organic solvents. Supercritical nitrogen has a lower extractive capacity for organic solvents than does supercritical carbon dioxide (the most commonly used primary supercritical fluid), but is also miscible with it. Supercritical helium can also be used as a secondary fluid, having almost zero capacity for most commonly used organic solvents. However, supercritical helium is much more expensive than supercritical nitrogen, and for that reason may not be so preferred.

The amount of secondary fluid co-introduced into the particle formation vessel (in other words, its pressure and flow rate relative to those of the other fluids being introduced) will depend on many factors, including the nature of the substance, vehicle and primary supercritical fluid, the concentration of the solution or suspension and the flow rates of the solution or suspension and the primary supercritical fluid. All of these factors affect how quickly the solution/suspension becomes supersaturated when the primary supercritical fluid extracts the vehicle from it, and hence how rapidly particle formation occurs.

For these reasons, the amount of secondary fluid used is best defined in a functional manner. It is preferably added in an amount sufficient to eliminate, at least substantially, blocking of the fluid inlet means, and/or of one or more of the supply lines used to supply fluids to the inlet means, as particle formation occurs. "Blocking" may generally be taken to mean the build-up of solids in a fluid line, which has the effect of restricting the usable diameter of the line by at least 5%, usually at least 25% or at least 50% and, in extreme cases, by 75% or more. The secondary fluid may be added in an amount sufficient only to eliminate "total" blockages, ie, of about 90% or more of the usable line diameter, which blockages can often lead to apparatus shutdown as pressure safety devices are triggered into operation.

The period over which such blockages are prevented, using the present invention, may be as long or short as desired. Ideally, the blockage reduction/elimination is effective throughout the entire, or substantially the entire, particle formation process. However, the important thing is that the extent and/or the frequency of blockage be less, using the method of the invention, than when carrying out the same process but without the secondary fluid.

In practical terms, the amount of secondary fluid needed to eliminate blockages might be expressed as an amount sufficient to reduce, preferably substantially to eliminate, pressure fluctuations in the fluid inlet means and/or in one or more of the supply lines used to supply fluids to the inlet means. The term "pressure fluctuations" here means fluctuations of greater than 30% of the relevant baseline fluid pressure, preferably of greater than 10%, more preferably of greater than 5%. In the case of fluctuations in the fluid inlet means, the baseline pressure, over which the fluctuations are measured, may be taken as that of the primary supercritical fluid.

Such pressure fluctuations occur (in addition to the relatively small background fluctuations caused by fluid pumps) when the inlet becomes blocked, the blockage clears, and it subsequently becomes blocked again. They can be measured in conventional ways, for instance using a standard pressure transducer to monitor the pressure in the inlet means or, more conveniently, in one or more of the fluid supply lines, such as the supply line for the solution/suspension. So long as the amount of secondary fluid used is just sufficient to eliminate these pressure fluctuations, one can be sure that little or no blockage is occurring. Naturally, amounts less than that may also be used, but with a smaller effect. Greater amounts may also be used, but care must be taken not to use so great an amount as to lose control over the physical characteristics of the particulate product formed. Also, the amount should not be so great that, under the operating conditions used, the secondary fluid is able to extract the vehicle to any appreciable extent.

The secondary fluid is preferably added in an adjustable flow. In this way, the flow rate of the secondary fluid can be controlled so as to ensure that the inlet means does not block, whilst also retaining control over the final product. The method of the invention can thus also involve monitoring the pressure in the fluid inlet means and/or in one or more of the fluid supply lines, and adjusting the flow of the secondary fluid to reduce or eliminate pressure fluctuations. The invention may then easily be used for producing a wide variety of substances in particulate form, from solutions or suspensions of a wide range of concentrations.

The secondary fluid is introduced into the particle formation vessel in the flow of the primary supercritical fluid, upstream of its point of contact with the solution or suspension. It may additionally be introduced with the solution/suspension. A convenient way of achieving such mixing is by using a-T-connection in the relevant fluid supply line(s), so that for instance the primary supercritical fluid and the secondary fluid mix upstream, of the fluid inlet means, and contact the solution or suspension together.

The method by which the solution or suspension and the primary supercritical fluid are co-introduced and contact one another to cause particle formation is preferably the method known as SEDS, or a modified version thereof, such as is described in WO-95/01221 or WO-96/00610. The present invention is particularly useful in combination with the SEDS process, to prevent blockage of the fluid inlet means when particle formation would otherwise occur too rapidly.

In the SEDS process, the solution or suspension and the primary supercritical fluid are co-introduced into the particle formation vessel in such a way that the primary supercritical fluid itself serves to disperse the solution or suspension, at the same time as it extracts the vehicle from it. This provides a very high degree of control over the particles formed.

When carrying out SEDS, the fluid inlet means is typically a co-axial nozzle having two or more passages, of the form described in WO-95/01221 and WO-96/00610. Such nozzles tend to have relatively narrow outlets, and it is at these outlets that the fluids contact one another and particle formation occurs. They are thus particularly prone to blockages, which the method of the present invention may be used to alleviate.

Any other modifications to the SEDS process, described in either WO-95/01221 or WO-96/00610 or indeed any other available literature, may also generally be combined with the method of the present invention.

In the invention, the relative flow rates of the fluids co-introduced into the particle formation vessel may be used to control the size and size distribution of the particles formed, for instance when using the primary supercritical fluid to disperse the solution or suspension as in SEDS. Preferably, the flow rate of the primary supercritical fluid is much higher than that of the solution or suspension - this leads to the formation of generally smaller fluid elements (eg droplets) of the solution or suspension, and hence relatively small particles having a narrow size distribution are formed when the primary supercritical fluid extracts the vehicle from the fluid elements.

The flow rate of the secondary fluid, relative to that of the primary supercritical fluid, will also depend on the materials involved and the extent of the blockage which it is intended to overcome. The secondary fluid flow rate might typically represent between about 0.05 and 0.8 mole fraction, preferably between about 0.25 and 0.5 mole fraction, of the sum of the primary and secondary fluid flows. These proportions would be suitable, for instance, when using supercritical nitrogen as the secondary fluid and supercritical carbon dioxide as the primary supercritical fluid.

The fluids which are co-introduced into the particle formation vessel are ideally made to flow in a smooth, continuous and preferably substantially pulse-less manner. This helps prevent draw-back of fluids into the inlet means, which could also lead to particle precipitation in undesirable locations and blocking of the apparatus. Conventional apparatus may be used to ensure such a fluid flow.

According to a sesond aspect of the present invention, there is provided a particulate product formed using the method of the first aspect.

The invention will now be described by way of example only, with reference to the accompanying illustrative drawings, of which:-
Fig. 1 is a schematic illustration of apparatus which may be used to carry out a method in accordance with the present invention;
Fig. 2 shows, also schematically, part of a fluid inlet means which may be used in the apparatus of Fig. 1;
Figs. 3-5 are schematic illustrations of alternative types of particle formation apparatus, that of Fig. 3 being of use to carry out the method of the invention; and
Figs. 6-13 are plots of pressure fluctuations observed in the fluid inlet nozzle in the experiments described below.

### Detailed Description

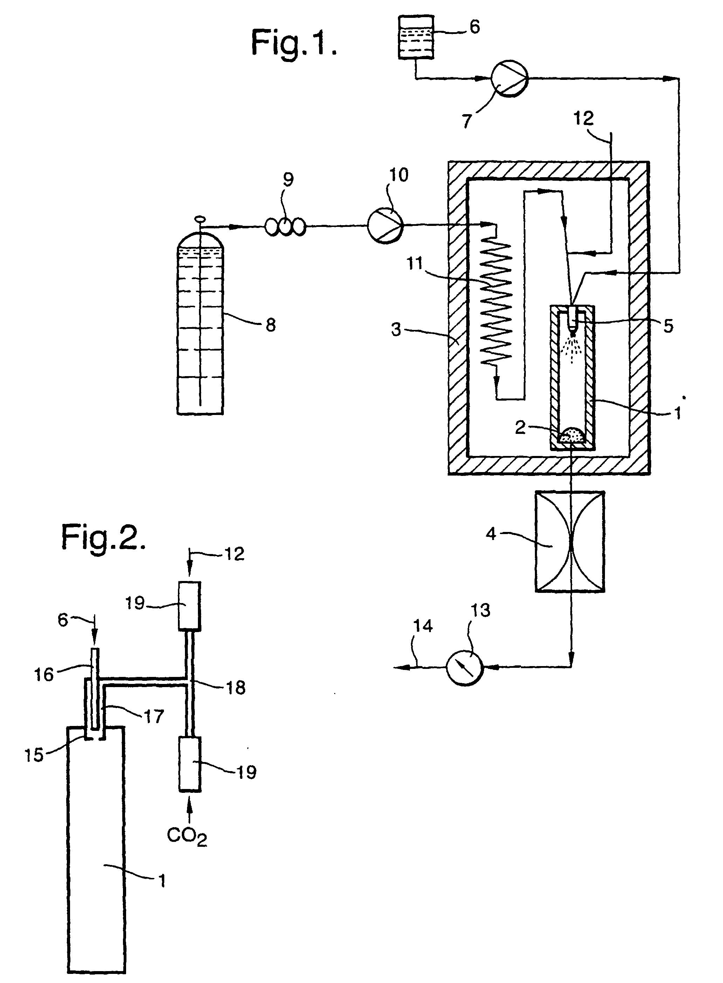

The apparatus of Fig. 1 includes a particle formation vessel 1 containing a particle retaining device (such as a filter or cyclone) 2. The temperature inside vessel 1 is controlled by means of the surrounding oven 3, and its internal pressure by means of the back pressure regulator 4. Both temperature and pressure are carefully controlled so as to maintain supercritical conditions within the vessel 1 at all times during its operation. Fluids are introduced into the vessel via any suitable fluid inlet, represented here by the nozzle 5.

In use, a solution or suspension 6 of a substance of interest in an appropriate vehicle is introduced, via pump 7 and the nozzle 5, into the particle formation vessel. A primary supercritical fluid (such as supercritical carbon dioxide) is also introduced via the nozzle 5 - in the case illustrated, carbon dioxide from source 8 passes through a cooler 9, pump 10 and heat exchanger 11 to convert it to its supercritical state before it enters the nozzle.

A secondary fluid 12, such as supercritical nitrogen, is introduced into the primary supercritical fluid supply line upstream of the nozzle, so that both supercritical fluids contact the solution/suspension 6, and enter the particle formation vessel, together.

Particle formation occurs in the vessel 1, preferably at the outlet of the nozzle 5, and the particles formed are collected in the retaining device 2. The fluids can be removed, via the back pressure regulator 4, flow meter 13 and vent 14.

Used with this apparatus is a pressure transducer (not shown), set up to monitor pressure fluctuations in the supply line connecting the nozzle to the solution/suspension source or to the primary supercritical fluid source. The transducer will preferably be placed in the supply line for the solution/suspension, which is essentially non-compressible. The transducer will then provide a trace indicating pressure variations at the nozzle.

Fig. 2 shows in more detail a fluid inlet device and associated apparatus, which may be used as part of the apparatus of Fig. 1. In this case the fluid inlet comprises a two-passage co-axial nozzle 15. The solution/suspension 6 may be introduced through the inner nozzle passage 16, and the primary supercritical fluid and secondary fluid through the outer passage 17.

The primary and secondary fluids are mixed using a T-connector 18 upstream of the nozzle. The components labelled 19 are pulse dampeners and heat exchangers for the two fluid flows, used to ensure smooth fluid flows and to bring the primary and secondary fluids to similar temperatures at the nozzle inlet.

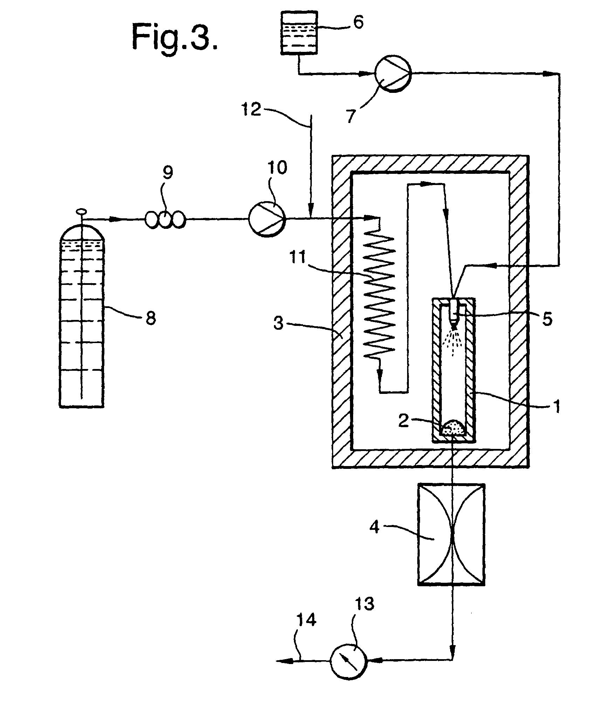

In the alternative apparatus of Fig. 3 (in which the same reference numerals are used for analogous parts), the secondary fluid 12 is introduced into the primary supercritical fluid supply line upstream of the heat exchanger 11. Both fluids are brought together to the correct operating temperature. This minimises the disturbance caused by the introduction of the secondary fluid, which could otherwise affect the temperature and pressure of the incoming, primary supercritical fluid.

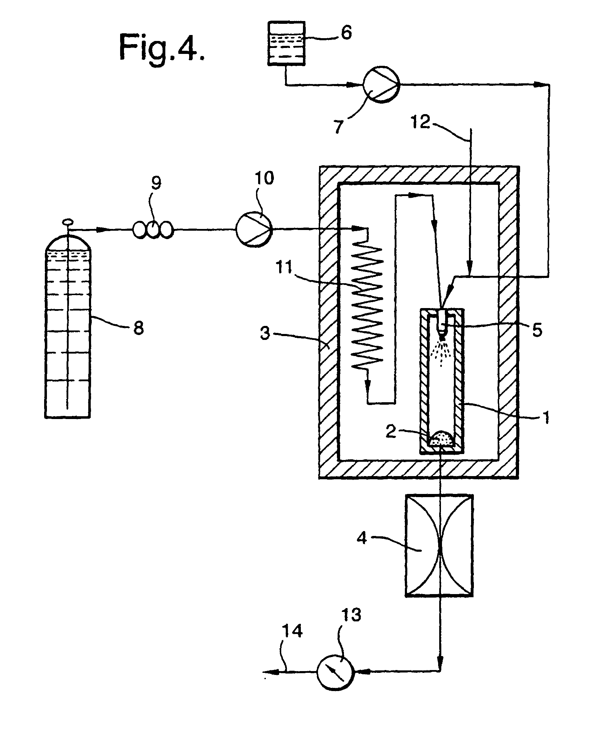

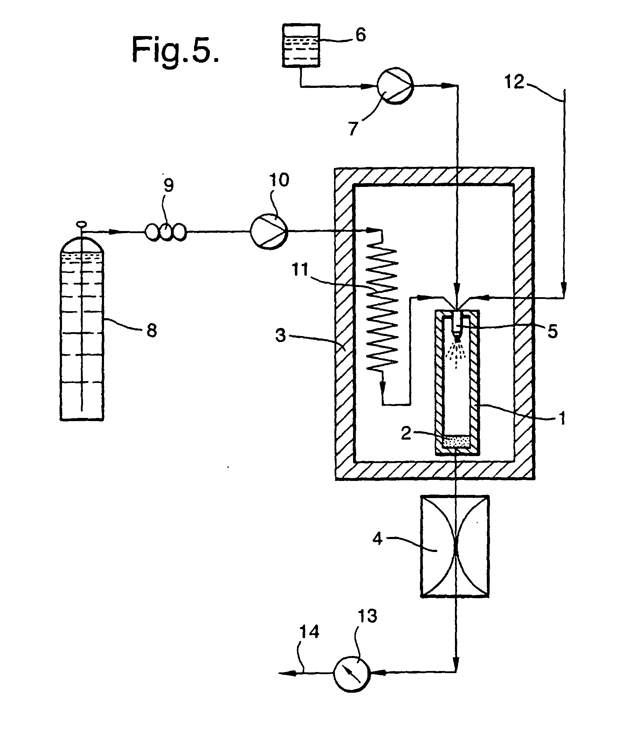

Fig. 4 shows how a secondary fluid may be introduced into the solution/suspension supply line, again upstream of the fluid inlet nozzle 5. Fig. 5 shows how it may be introduced into the nozzle separately from the other fluids, only contacting them just upstream of, or at, the point of particle formation.

In connection with Figs. 1-5, it should be pointed out that when using low boiling point gases such as nitrogen, difficulties can arise in pumping the liquefied gases. Cryogenic pumps do exist, but for the laboratory scale it can be more convenient to use cylinders providing a 230 bar or similar service. The gases can simply be vented from their higher pressure cylinders into the particle formation vessel 1 which is at.a lower pressure. Their flow can be controlled by a needle valve. However, gas boosters can also be used to ensure flow consistency and to achieve pressures higher than those of conventional gas cylinders.

### Experimental Examples

Experiments were carried out using apparatus of the type illustrated in Figs. 1 and 2. Carbon dioxide was used as the primary supercritical fluid, and supercritical nitrogen as the secondary fluid. The SEDS process was used to produce particles of paracetamol, from solutions of paracetamol in ethanol. Relatively high paracetamol concentrations were used, at which nozzle blockages often occur (it should be noted that an 8% w/v solution of paracetamol in ethanol at 25 °C is close to saturation).

During the experiments different amounts of the secondary fluid were introduced into the system, to assess its effect on nozzle blockages. In the following description, secondary fluid flow rates are quoted in litres per minute of gas under ambient conditions as measured at the vent line beyond the back pressure regulator.

All runs were carried out using a two-passage co-axial nozzle having a 200 micrometer nozzle tip.

The first two runs were to investigate the effect of sonication in reducing blockages - this is a conventional way of dealing with the problem.

In each run, the operating pressure was 90 bar and the operating temperature 60°C. The flow rate of the supercritical carbon dioxide was 9 ml/min, and that of the paracetamol solution was 0.1 ml/min.

Table 1 below summaries the eight runs carried out.

**Table 1**

| **Run number** | **Secondary Fluid flow (1/min)*** | **Secondary Fluid** | **Paracetamol concentration (%w/v)** |
|---|---|---|---|
| 1 (with sonication) | n/a | n/a | 1 |
| 2 (without sonication) | n/a | n/a | 1 |
| 3 | 3 | N₂ | 1 |
| 4 | 3 | N₂ | 8 |
| 5 | n/a | n/a | 8 |
| 6 | 5 | N₂ | 8 |
| 7 | 10 | N₂ | 8 |
| 8 | 5 | He | 8 |

| | | | |
|---|---|---|---|
| * Flow measured at atmospheric pressure using a rotameter calibrated for CO₂. | | | |

### Results

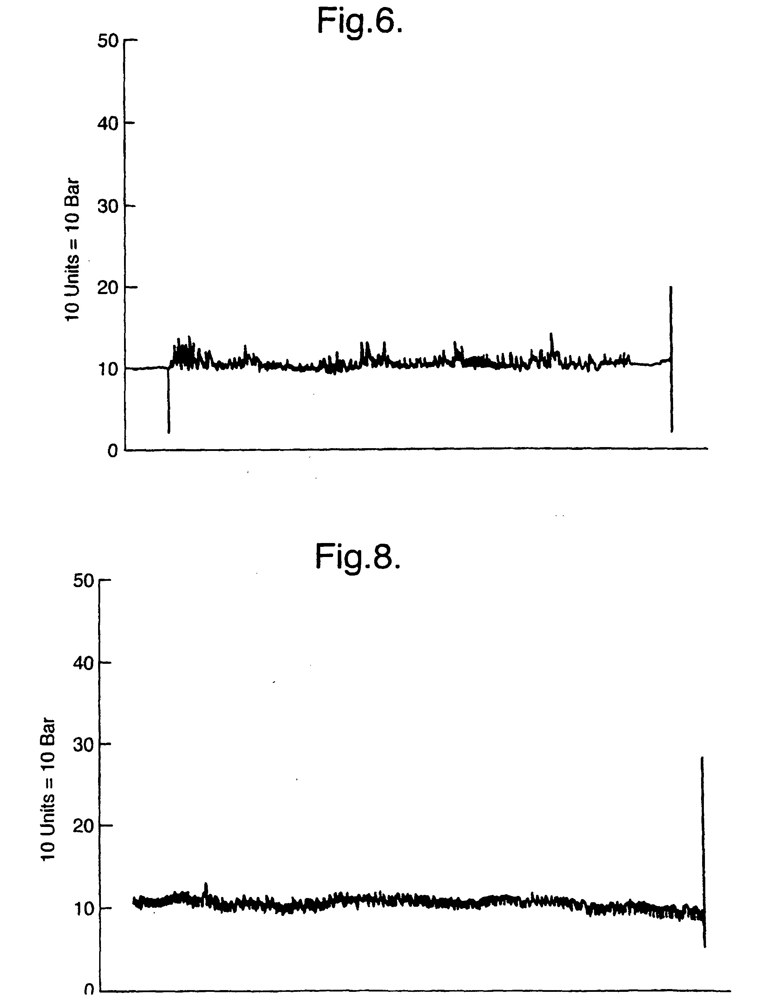

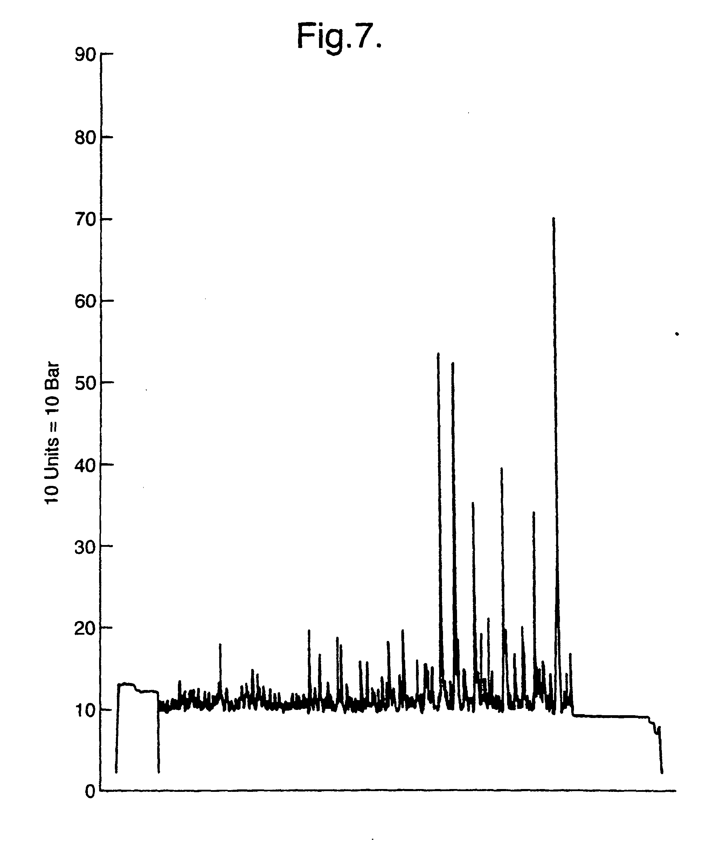

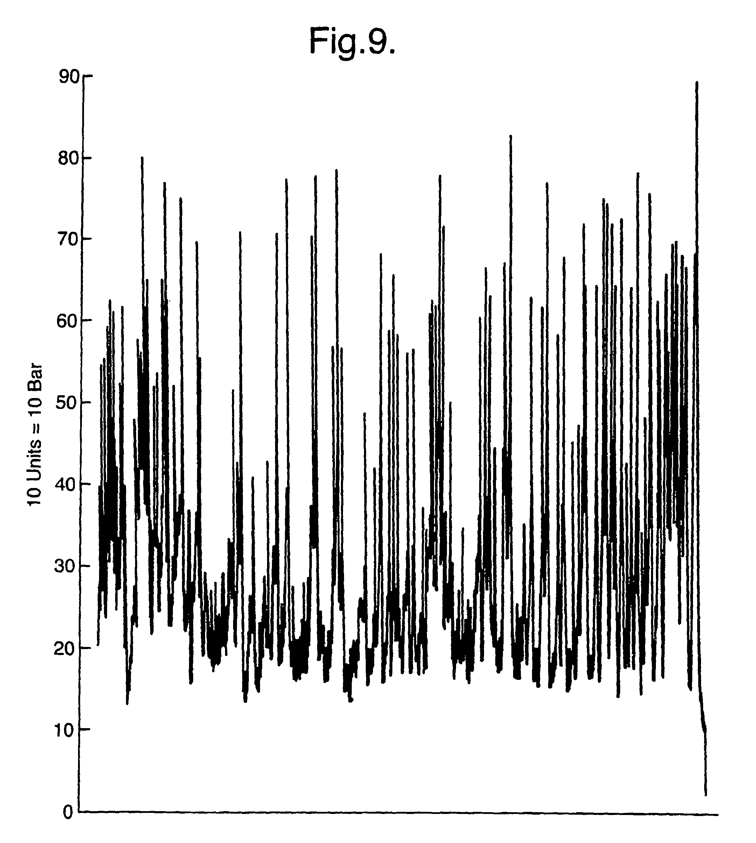

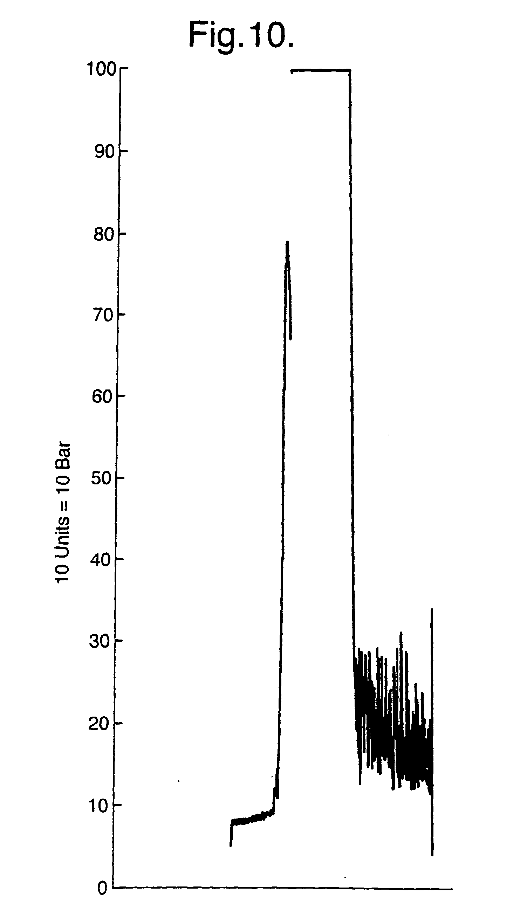

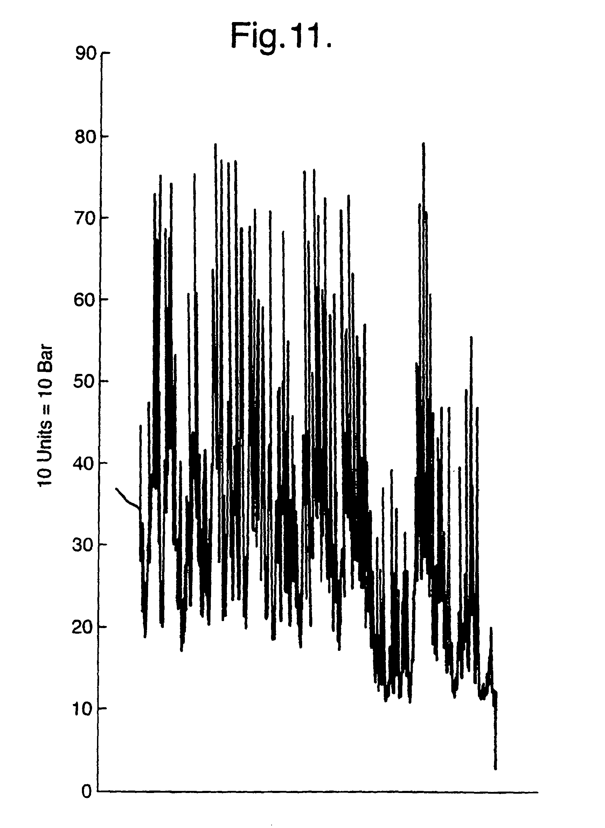

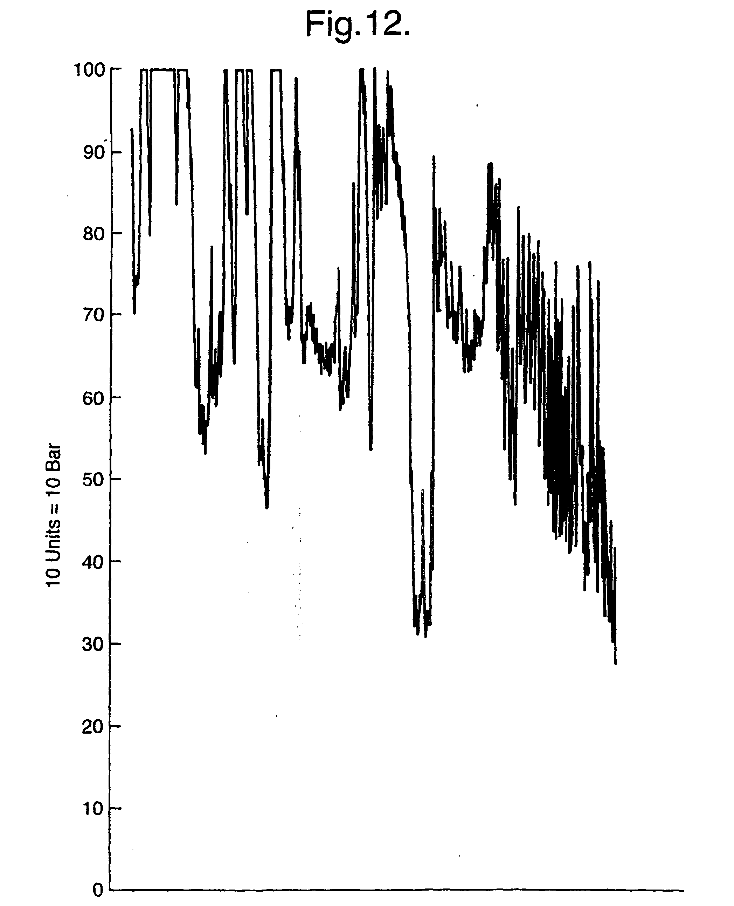

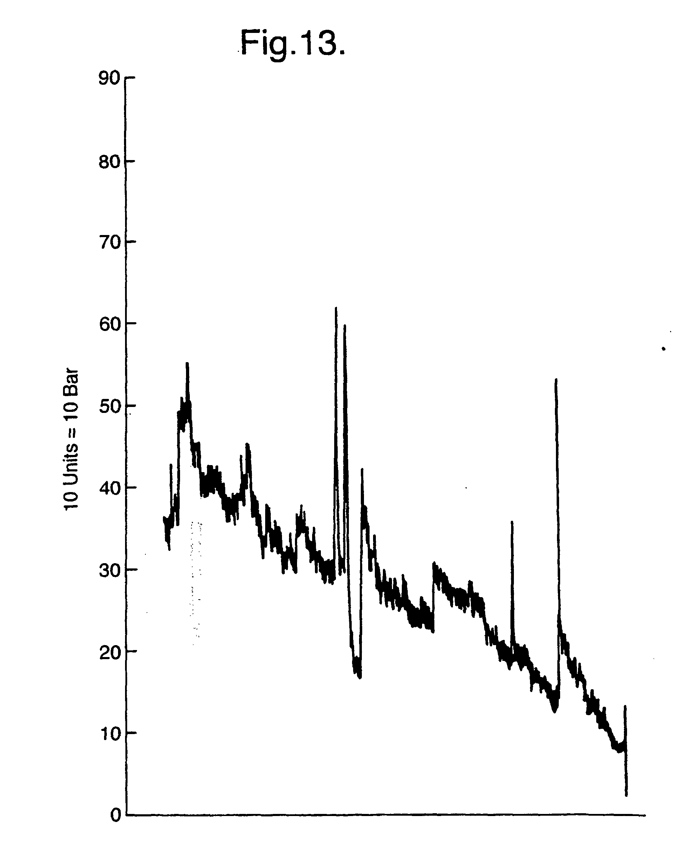

The pressure fluctuations in the nozzle, observed using the pressure transducer, are plotted in Figs. 6-13 for experimental runs 1-8 respectively. Table 2 below contains a summary of these results. The mean and peak pressure fluctuations shown are the pressures reached *above* the pressure in the particle formation vessel 1. Spikes in the traces indicate continual blocking and clearing of the nozzle. The background noise in all runs is due to the relatively small pressure variations produced by the reciprocating solution/suspension pumps.

**Table 2**

| **Run number** | **Mean pressure fluctuation (bar)** | **Peak pressure fluctuation (bar)** |
|---|---|---|
| 1 | 1-2 | 4 |
| 2 | 5 | 60 |
| 3 | 1 | 1 |
| 4 | 35 | 70 |
| 5 | overpressure | overpressure |
| 6 | 25 | 60 |
| 7 | 50-60 | >100 |
| 8 | 10 | 50 |

### Discussion

Firstly, runs 1 and 2 demonstrate that the use of ultrasonics can reduce the incidence of nozzle blockage for 1% w/v paracetamol in ethanol solutions. This can be attributed to a "descaling" effect of the ultrasonics, ie, particles can be shaken free of an area of blockage.

Runs 3 and 4 show how the use of supercritical nitrogen at a relatively low flow rate (3 l/min) can greatly reduce nozzle blockages, even (run 4) with an 8% paracetamol solution. Run 3, using only a 1% paracetamol solution, showed no nozzle blockage at all. By comparison, run 5 (8% paracetamol solution, but no secondary fluid) suffered from blockage as soon as the paracetamol solution entered the nozzle, causing the fluid pumps to reach their pressure cut-off limit of 500 bar and aborting the run without particle formation. The higher paracetamol concentration of course increases the rate of particle formation, since less solvent needs to be extracted before the solution becomes supersaturated.

At a paracetamol concentration of 8% w/v run 4 (3 l/min of supercritical nitrogen) showed a reduced incidence of nozzle blockage and run 6 (5 l/min of supercritical nitrogen) an even greater reduction.

Run 7 demonstrates the effect of increasing the flow of the secondary fluid by too much. At 10 l/min of supercritical nitrogen, nozzle blockages actually become greater than at lower nitrogen flow rates. This is because, although supercritical nitrogen is much less able than supercritical carbon dioxide to extract ethanol from the paracetamol solution, it still has some extractive properties. Previous experiments, using nitrogen gas as the sole extracting fluid, have indicated that a flow rate of 10 1/min is sufficient to produce particles using a paracetamol solution flow of 0.1 ml/min. This is what might be occurring in run 7. In other words, the nitrogen may be supplementing the extractive properties of the carbon dioxide and also removing solvent, to an extent sufficient to cause excessive nozzle blocking.

Run 8 demonstrates that this problem can be overcome by using an alternative secondary fluid with a yet lower capacity for the ethanol solvent. In this case, supercritical helium replaced supercritical nitrogen - helium has an extractive capacity for almost all substances of close to zero and can be considered as completely inert. Run 8 replicates run 6 except with helium substituted for the nitrogen. Pressure fluctuations are greatly reduced.

Analysis of the paracetamol particles formed (for instance using a scanning electron microscope) suggests that particle formation is only marginally affected by the co-introduction of a secondary fluid. In particular it should be borne in mind that the method of the present invention makes it possible to form useable particulate products in situations where otherwise there would be too much blockage for particle formation to proceed.

## Claims

1. A method for forming particles of a substance, the method comprising:-
(a) preparing a solution or suspension of the substance in a vehicle;
(b) introducing the solution or suspension into a particle formation vessel via a fluid inlet means; and
(c) introducing a primary supercritical fluid, capable of acting as an anti-solvent for the substance, into the particle formation vessel, under conditions which allow the supercritical fluid to extract the vehicle from the solution or suspension and hence cause the formation of particles of the substance;
**characterised in that** a secondary fluid is introduced into the particle formation vessel in the flow of the primary supercritical fluid, upstream of the point of contact between the primary supercritical fluid and the solution or suspension, the secondary fluid having a lower capacity for extracting the vehicle than that of the primary supercritical fluid.

2. A method according to claim 1, wherein the primary supercritical fluid and the solution or suspension are co-introduced into the particle formation vessel in such a way that the primary supercritical fluid itself serves to disperse the solution or suspension, at the same time as it extracts the vehicle from it, the fluids being co-introduced through a fluid inlet means which allows them both to enter the vessel at the same or substantially the same point, which is also the same as, or substantially the same as, the point where they meet and at which the primary supercritical fluid serves to disperse the solution or suspension.

3. A method according to claim 1 or claim 2, wherein the capacity of the secondary fluid for extracting the vehicle is less than 30 % as great as that of the primary supercritical fluid.

4. A method according to any one of the preceding claims, wherein the secondary fluid is a supercritical fluid.

5. A method according to claim 4, wherein the primary supercritical fluid is supercritical carbon dioxide and the secondary fluid is supercritical nitrogen.

6. A method according to any one of the preceding claims, wherein the secondary fluid is introduced in an adjustable flow, the method additionally involving monitoring the pressure in the fluid inlet means and/or in one or more fluid supply lines, and adjusting the flow of the secondary fluid to reduce or eliminate pressure fluctuations.

7. A method according to any one of the preceding claims, wherein the secondary fluid is introduced into the flow of both the primary supercritical fluid and the solution or suspension, upstream of their point of contact with one another.

8. A method according to any one of the preceding claims, wherein the flow rate of the supercritical fluid represents between 0.05 and 0.8 mole fraction of the sum of the primary and secondary fluid flows.

9. Use, in a method for forming particles of a substance which comprises:-
(a) preparing a solution or suspension of the substance in a vehicle;
(b) introducing the solution or suspension into a particle formation vessel via a fluid inlet means; and
(c) introducing a primary supercritical fluid, capable of acting as an anti-solvent for the substance, into the particle formation vessel, under conditions which allow the supercritical fluid to extract the vehicle from the solution or suspension and hence cause the formation of particles of the substance,
of a secondary fluid which has a lower capacity for extracting the vehicle than that of the primary supercritical fluid, the secondary fluid being introduced into the particle formation vessel in the primary supercritical fluid flow, upstream of the point of contact between the primary supercritical fluid and the solution or suspension, for the purpose of eliminating, at least substantially, blocking of the fluid inlet means, and/or of one or more of the supply lines used to supply fluids to the inlet means, as particle formation occurs.

10. Use, in a method for forming particles of a substance which comprises:-
(a) preparing a solution or suspension of the substance in a vehicle;
(b) introducing the solution or suspension into a particle formation vessel via a fluid inlet means; and
(c) introducing a primary supercritical fluid, capable of acting as an anti-solvent for the substance, into the particle formation vessel, under conditions which allow the supercritical fluid to extract the vehicle from the solution or suspension and hence cause the formation of particles of the substance,
of a secondary fluid which has a lower capacity for extracting the vehicle than that of the primary supercritical fluid, the secondary fluid being introduced into the particle formation vessel in the flow of the primary supercritical fluid, upstream of the point of contact between the primary supercritical fluid and the solution or suspension, for the purpose of reducing pressure fluctuations in the fluid inlet means and/or in one or more of the supply lines used to supply fluids to the fluid inlet means.

## Patentansprüche

1. Verfahren zur Bildung von Teilchen einer Substanz, wobei das Verfahren umfasst:
(a) Herstellen einer Lösung oder Suspension der Substanz in einem Vehikel,
(b) Einleiten der Lösung oder Suspension in einen Teilchenbildungsbehälter über eine Fluid-Einlasseinrichtung und
(c) Einleiten eines primären überkritischen Fluids, das in der Lage ist als Nicht-Lösungsmittel für die Substanz zu wirken, in den Teilchenbildungsbehälter unter Bedingungen, die es ermöglichen, dass das überkritische Fluid das Vehikel aus der Lösung oder Suspension extrahiert und daher die Bildung von Teilchen der Substanz verursacht,
**dadurch gekennzeichnet, dass** ein sekundäres Fluid in den Teilchenbildungsbehälter in dem Strom des primären überkritischen Fluids stromaufwärts von der Kontaktstelle zwischen dem primären überkritischen Fluid und der Lösung oder Suspension eingeleitet wird, wobei das sekundäre Fluid ein geringeres Vermögen zur Extraktion des Vehikels als das primäre überkritische Fluid aufweist.

2. Verfahren nach Anspruch 1, wobei das primäre überkritische Fluid und die Lösung oder Suspension in einer solchen Weise gemeinsam in den Teilchenbildungsbehälter eingeleitet werden, dass das primäre überkritische Fluid selbst zur gleichen Zeit zur Verteilung der Lösung oder Suspension dient, zu der es das Vehikel daraus extrahiert, wobei die Fluide über eine Fluid-Einlasseinrichtung gemeinsam eingeleitet werden, die es ermöglicht, dass beide an der gleichen Stelle oder im wesentlichen an der gleichen Stelle in den Behälter eintreten, welche auch die gleiche oder im wesentlichen die gleiche wie die Stelle ist, an der sie sich treffen und an der das primäre überkritische Fluid zur Verteilung der Lösung oder Suspension dient.

3. Verfahren nach Anspruch 1 oder Anspruch 2, wobei das Vermögen des sekundären Fluids zur Extraktion des Vehikels weniger als 30% so groß ist wie das des primären überkritischen Fluids.

4. Verfahren nach irgendeinem der vorhergehenden Ansprüche, wobei das sekundäre Fluid ein überkritisches Fluid ist.

5. Verfahren nach Anspruch 4, wobei das primäre überkritische Fluid überkritisches Kohlendioxid ist und das sekundäre Fluid überkritischer Stickstoff ist.

6. Verfahren nach irgendeinem der vorhergehenden Ansprüche, wobei das sekundäre Fluid in einem regelbaren Strom eingeleitet wird, wobei das Verfahren zusätzlich die Überwachung des Drucks in der Fluid-Einlasseinrichtung und/oder in einer oder mehreren Fluid-Zufuhrleitungen und das Regeln des Stroms des sekundären Fluids zur Verringerung oder Beseitigung von Druckfluktuationen beinhaltet.

7. Verfahren nach irgendeinem der vorhergehenden Ansprüche, wobei das sekundäre Fluid in den Strom von sowohl dem primären überkritischen Fluid als auch der Lösung oder Suspension stromaufwärts von ihrer Kontaktstelle miteinander eingeleitet wird.

8. Verfahren nach irgendeinem der vorhergehenden Ansprüche, wobei der Durchsatz des überkritischen Fluids einen Molbruch zwischen 0,05 und 0,8 von der Summe der Ströme des primären und sekundären Fluids darstellt.

9. Verwendung in einem Verfahren zur Bildung von Teilchen einer Substanz, welches umfasst:
(a) Herstellen einer Lösung oder Suspension der Substanz in einem Vehikel,
(b) Einleiten der Lösung oder Suspension in einen Teilchenbildungsbehälter über eine Fluid-Einlasseinrichtung und
(c) Einleiten eines primären überkritischen Fluids, das in der Lage ist als Nicht-Lösungsmittel für die Substanz zu wirken, in den Teilchenbildungsbehälter unter Bedingungen, die es ermöglichen, dass das überkritische Fluid das Vehikel aus der Lösung oder Suspension extrahiert und damit die Bildung von Teilchen der Substanz verursacht,
von einem sekundären Fluid, das ein geringeres Vermögen zur Extraktion des Vehikels als das primäre überkritische Fluid aufweist, wobei das sekundäre Fluid in den Teilchenbildungsbehälter in dem Strom des primären überkritischen Fluids stromaufwärts von der Kontaktstelle zwischen dem primären überkritischen Fluid und der Lösung oder Suspension eingeleitet wird, um das Verstopfen der Fluid-Einlasseinrichtung und/oder von einer oder mehreren der Zufuhrleitungen, die für die Zuführung von Fluiden zur Einlasseinrichtung verwendet werden, zumindest im wesentlichen zu beseitigen, wenn Teilchenbildung stattfindet.

10. Verwendung in einem Verfahren zur Bildung von Teilchen einer Substanz, welches umfasst:
(a) Herstellen einer Lösung oder Suspension der Substanz in einem Vehikel,
(b) Einleiten der Lösung oder Suspension in einen Teilchenbildungsbehälter über eine Fluid-Einlasseinrichtung und
(c) Einleiten eines primären überkritischen Fluids, das in der Lage ist als Nicht-Lösungsmittel für die Substanz zu wirken, in den Teilchenbildungsbehälter unter Bedingungen, die es ermöglichen, dass das überkritische Fluid das Vehikel aus der Lösung oder Suspension extrahiert und damit die Bildung von Teilchen der Substanz verursacht,
von einem sekundären Fluid, das ein geringeres Vermögen zur Extraktion des Vehikels als das primäre überkritische Fluid aufweist, wobei das sekundäre Fluid in den Teilchenbildungsbehälter in dem Strom des primären überkritischen Fluids stromaufwärts von der Kontaktstelle zwischen dem primären überkritischen Fluid und der Lösung oder Suspension eingeleitet wird, um Druckfluktuationen in der Fluid-Einlasseinrichtung und/oder in einer oder mehreren der Zufuhrleitungen, die zur Zuführung von Fluiden zur Fluid-Einlasseinrichtung verwendet werden, zu verringern.

## Revendications

1. Procédé pour la formation de particules d'une substance, le procédé comprenant :
(a) la préparation d'une solution ou suspension de la substance dans un véhicule ;
(b) l'introduction de la solution ou suspension dans un récipient de formation de particules par l'intermédiaire d'un moyen d'admission de fluide ; et
(c) l'introduction d'un fluide supercritique primaire, capable d'agir comme un antisolvant pour la substance, dans le récipient de formation de particules, dans des conditions qui permettent que le fluide supercritique extraie le véhicule de la solution ou suspension et en conséquence qu'il induise la formation des particules de la substance ;
**caractérisé en ce qu'**un fluide secondaire est introduit dans le récipient de formation de particules dans l'écoulement du fluide supercritique primaire, en amont du point de contact entre le fluide supercritique primaire et la solution ou suspension, le fluide secondaire ayant une capacité pour l'extraction du véhicule inférieure à celle du fluide supercritique primaire.

2. Procédé selon la revendication 1, dans lequel le fluide supercritique primaire et la solution ou suspension sont co-introduits dans le récipient de formation de particules d'une façon telle que le fluide supercritique primaire serve lui-même à disperser la solution ou suspension, au même moment où il extrait le véhicule de celle-ci, les fluides étant co-introduits au moyen d'un moyen d'admission de fluide qui permet que les deux à la fois entrent dans le récipient au même ou sensiblement le même endroit, qui est également le même, ou sensiblement le même, que l'endroit où ils se rejoignent et où le fluide supercritique sert à disperser la solution ou suspension.

3. Procédé selon la revendication 1 ou 2, dans lequel la capacité du fluide secondaire pour l'extraction du véhicule est moins de 30 % aussi grande que celle du fluide supercritique primaire.

4. Procédé selon l'une quelconque des revendications précédentes, dans lequel le fluide secondaire est un fluide supercritique.

5. Procédé selon la revendication 4, dans lequel le fluide supercritique primaire est du dioxyde de carbone supercritique et le fluide secondaire est de l'azote supercritique.

6. Procédé selon l'une quelconque des revendications précédentes, dans lequel le fluide secondaire est introduit dans un écoulement ajustable, le procédé impliquant facultativement la surveillance de la pression dans le moyen d'admission de fluide et/ou dans une ou plusieurs lignes d'alimentation de fluide, et l'ajustement de l'écoulement du fluide secondaire pour réduire ou éliminer les fluctuations de pression.

7. Procédé selon l'une quelconque des revendications précédentes, dans lequel le fluide secondaire est introduit dans l'écoulement d'à la fois le fluide supercritique principal et la solution ou suspension, en amont de leur point de contact l'un avec l'autre.

8. Procédé selon l'une quelconque des revendications précédentes, dans lequel la vitesse d'écoulement du fluide supercritique représente entre 0,05 et 0,8 fraction molaire de la somme des débits des fluides primaire et secondaire.

9. Utilisation, dans un procédé pour la formation de particules d'une substance qui comprend :
(a) la préparation d'une solution ou suspension de la substance dans un véhicule ;
(b) l'introduction de la solution ou suspension dans un récipient de formation de particules par l'intermédiaire d'un moyen d'admission de fluide ; et
(c) l'introduction d'un fluide supercritique primaire, capable d'agir comme un antisolvant pour la substance, dans le récipient de formation de particules, dans des conditions qui permettent que le fluide supercritique extraie le véhicule de la solution ou suspension et en conséquence induise la formation des particules de la substance,
d'un fluide secondaire qui a une capacité d'extraction du véhicule inférieure à celle du fluide supercritique primaire, le fluide secondaire étant introduit dans le récipient de formation de particules dans l'écoulement du fluide supercritique primaire, en amont du point de contact entre le fluide supercritique primaire et la solution ou suspension, dans le but d'éliminer, au moins sensiblement, le blocage du moyen d'admission de fluide, et/ou d'une ou plusieurs des lignes d'alimentation utilisées pour alimenter en fluides le moyen d'admission, au fur et à mesure que la formation des particules se produit.

10. Utilisation, dans un procédé pour la formation de particules d'une substance qui comprend :
(a) la préparation d'une solution ou suspension de la substance dans un véhicule ;
(b) l'introduction de la solution ou suspension dans un récipient de formation de particules par l'intermédiaire d'un moyen d'admission de fluide ; et
(c) l'introduction d'un fluide supercritique primaire, capable d'agir comme un antisolvant pour la substance, dans le récipient de formation de particules, dans des conditions qui permettent que le fluide supercritique extraie le véhicule de la solution ou suspension et en conséquence induise la formation des particules de la substance,
d'un fluide secondaire qui a une capacité d'extraction du véhicule inférieure à celle du fluide supercritique primaire, le fluide secondaire étant introduit dans le récipient de formation de particules dans le débit du fluide supercritique primaire, en amont du point de contact entre le fluide supercritique primaire et la solution ou suspension, dans le but de réduire les fluctuations de pression dans le moyen d'admission de fluide et/ou dans une ou plusieurs des lignes d'alimentation utilisées pour alimenter en fluides le moyen d'admission de fluide.
